# EUROPÄISCHE PATENTSCHRIFT

(11) **EP 1 242 816 B1**
(45) Veröffentlichungstag und Bekanntmachung des Hinweises auf die Patenterteilung: **10.03.2004**
(21) Anmeldenummer: 00992208.9
(22) Anmeldetag: 20.12.2000
(51) Int. Cl.: G01N 30/48, B01J 41/06, B01J 39/06, B01J 20/32, C12N 15/10

(54) **CHROMATOGRAPHIEMATERIAL UND VERFAHREN UNTER VERWENDUNG DESSELBEN**
CHROMATOGRAPHY MATERIAL AND A METHOD USING SAME
MATERIAU CHROMATOGRAPHIQUE ET PROCEDES D'UTILISATION D'UN TEL MATERIAU

(30) Priorität: 23.12.1999 DE 19962577
(43) Veröffentlichungstag der Anmeldung: 25.09.2002
(73) Patentinhaber: Dr. Tittgen Biotechnologie, 32257 Bünde (DE)
(72) Erfinder: TITTGEN, Jochen, 32257 Bünde (DE)
(74) Vertreter: Draudt, Jutta
(86) Internationale Anmeldenummer: PCT/EP2000/013034
(87) Internationale Veröffentlichungsnummer: WO 2001/046686

(56) Entgegenhaltungen:
- EP-A- 0 276 138
- EP-A- 0 443 734
- EP-A- 0 649 853
- WO-A-91/05606
- WO-A-99/51734
- US-A- 5 843 312

## Beschreibung

Die vorliegende Erfindung betrifft ein Chromatographiematerial zur Trennung von Nukleinsäuregemischen und ein Verfahren zur Trennung von Nukleinsäuregemischen unter Verwendung dieses Chromatographiematerials.

Die Anwendung der Chromatographie sowie die dabei verwendeten Chromatographiematerialien sind auf dem Gebiet der Biochemie, Medizin, Pharmazie und Gentechnologie unerlässlich. Mit Hilfe von Chromatographiematerialien werden Biomoleküle, wie Nukleinsäuren und Proteine, rasch und systematisch aufgetrennt und isoliert. In der Molekularbiologie müssen oftmals aus einem natürlich vorkommenden Gemisch aus mehr als hundert verschiedenen Komponenten bestimmte Nukleinsäuren isoliert werden, welche in diesem Gemisch in Konzentrationen von weniger als 0,1 % enthalten sind. Die Anforderungen an ein chromatographisches Verfahren und das darin verwendete Chromatographiematerial umfassen daher zum einen die quantitative Isolierung der Nukleinsäuren und zum anderen die quantitative Abtrennung der Verunreinigungen, um somit die Nukleinsäure als molekulare Spezies bis zum Homogenität für die anschließende Analyse zu reinigen.

Bei den bekannten Chromatgraphieverfahren werden anorganische gekörnte Chromatographiematerialien mit definierten Korn- und Porengrößen eingesetzt, deren Oberfläche mit einem Silanisierungsreagenz zur Herstellung einer stationären Phase modifiziert ist. Die Reaktion mit einem Anionen- oder Kationenaustauscher bildenden Reagenz führt dann zum fertigen Chromatographiematerial.

So ist in der WO 91/05606 ein Trägermaterial für die Chromatographie beschrieben, das sich zur Trennung verschiedener Nukleinsäurearten eignet. Als Träger kommen Kieselgel, Aluminiumoxid, Titandioxid, poröses Glas oder Harze in Betracht. Das Trägermaterial soll eine Korngröße von 3 bis 500 µm mit einer Porengröße von etwa 10 bis 1000 nm und einer spezifischen Oberfläche von 5 bis 800 m²/g aufweisen. Die Oberfläche dieses körnigen Trägers wird mit Alkoxysilanen silaniert. So wird außerdem ein chromatographisches Trägermaterial auf Kieselgelbasis mit Anionenaustauschergruppen beschrieben, die durch Umsetzung des Alkoxysilans zusammen mit einem sekundären Hydroxylamin erhalten werden.
So werden auch gemäß der EP 0 104 210 Kieselgelmaterialien mit einer Korngröße von 3 bis 100 µm beschrieben, die eine Hohlraumgröße von 10 bis 1000 nm und eine spezifische Oberfläche von 5 bis 800 m²/g aufweisen. Diese werden dann mit einem Silanisierungsmittel oberflächenbehandelt und mit Ionenaustauscherfunktionen in der Chromatographie zur Trennung von Nukleinsäuren eingesetzt.

Schließlich ist aus der EP 0744025 ein Chromatographiematerial bekannt, bei dem ein Träger aus Kieselgel mit einem Silanisierungsreagenz umgesetzt ist, wobei Trägermaterialien gewählt werden, die einen Porendurchmesser von 4 bis 6 nm aufweisen.
Die Partikelgröße des Trägers beträgt 1 bis 500 µm.

EP 0 443 734 beschreibt Zirconiumfasern mit beschichteten oder modifizierten Oberflächen, die für chromatographische Anwendungen geeignet sind.

Bei den chromatographischen Trennungen von Nukleinsäuregemischen unter Verwendung der konventionellen körnigen Chromatographiematerialen hat sich herausgestellt, dass die Trennungen jeweils mit einem erheblichen Zeitaufwand durchgeführt werden mussten. Wenn beispielsweise eine für die Chromatographie bestimmte Säule mit einem modifizierten Kieselgel als Träger verwendet wurde, so war damit zu rechnen, dass unter Gravity-flow-Bedingungen eine Adsorptionszeit der Nukleinsäuren an den Träger von mindestens 20 Minuten in Kauf genommen werden musste.

Die bisher bekannten Chromatographiematerialien weisen außerdem eine definierte Porosität auf, da nach herrschender Meinung nur eine poröse, d. h. vergrößerte Oberfläche des Trägers eine ausreichende Beladung mit Ionenaustauscher gewährleistet. Diese Porosität hat aber den Nachteil, dass die Qualität der Trennung der Nukleinsäure nicht optimal ist. Das ist darauf zurückzuführen, dass während der Trennung andere im Gemisch befindliche Substanzen, wie RNAs und Proteine, in die Poren diffundieren und damit den Trennvorgang ungünstig beeinflussen.

Der vorliegenden Erfindung lag somit die Aufgabe zugrunde, ein Chromatographiematerial zur Verfügung zu stellen, das innerhalb kürzester Zeit eine Trennung von Nukleinsäuregemischen vornehmen kann, wobei gleichzeitig eine hervorragende Auflösung der Nukleinsäuregemische und damit verbundene Reinheit der zu isolierenden Nukleinsäuren erreicht werden kann.

Es ist weiterhin Aufgabe der vorliegenden Erfindung, ein Verfahren zur Verfügung zu stellen, mit dem in kürzester Zeit ein Nukleinsäuregemisch mit höchster Auflösung und Reinheit der Einzelkomponenten aufgetrennt werden kann.

Diese Aufgaben werden mit den Merkmalen des Patentanspruchs 1 und des Patentanspruchs 12 gelöst.

Die vorliegende Erfindung betrifft ein Chromatographiematerial zur Trennung von Nukleinsäuregemischen mit einem Träger und darauf aufgebrachten Ionenaustauscherfunktionen, wobei der Träger ein fasriges Material umfasst.

Die Unteransprüche betreffen bevorzugte Ausführungsformen des erfindungsgemäßen Chromatographiematerials.

Die Erfindung betrifft ein Verfahren zur Trennung von Nukleinsäuregemischen mit dem erfindungsgemäßen Chromatographiematerial, wobei die chromatographische Trennung der Nukleinsäuren mittels Einwirken einer Kraft durchgeführt wird.

Die Unteransprüche betreffen bevorzugte Ausführungsformen des erfindungsgemäßen Verfahrens.

Schließlich betrifft die Erfindung einen Kit zur Trennung von Nukleinsäuregemischen, das ein Chromatographiematerial gemäß der vorliegenden Erfindung enthält. Das Kit umfasst das erfindungsgemäße Chromatographiematerial in der gewünschten Anordnung zusammen mit entsprechenden Puffern zur Durchführung der chromatographischen Trennung von Nukleinsäuregemischen mit dem Chromatographiematerial.

Die Erfindung wird anhand der Figuren erläutert. Es zeigen:
- Fig. 1: ein Beispiel für eine Säule mit dem erfindungsgemäßen Chromatographiematerial;
- Fig. 2: die Auftrennung von Nukleinsäuregemischkomponenten auf einem Agarosegel unter Verwendung des erfindungsgemäßen Chromatographiematerials.;
- **Fig. 3**: die Auftrennung von Nukleinsäuregemischkomponenten auf einem Agarosegel unter Verwendung eines Chromatographiematerials des Standes der Technik und
- **Fig. 4**: die Auftrennung von Nukleinsäuregemischkomponenten auf einem Agarosegel unter Verwendung eines Chromatographiematerials des Standes der Technik.

Es hat sich erfindungsgemäß überraschenderweise herausgestellt, dass eine wesentlich verbesserte Trennkapazität erreicht werden kann, wenn der Träger des Chromatographiematerials ein fasriges Material umfasst, dessen Oberfläche nicht oder nur unwesentlich vergrößert ist. Entgegen der herrschenden Meinung kann das erfindungsgemäße Chromatographiematerial mit einer entsprechenden Menge an funktionellen Ionenaustauschergruppen beladen werden, um eine ausgezeichnete Trennkapazität zu gewährleisten.

Die spezifische Oberfläche des Trägers liegt im Bereich von 0,05 bis 50 m²/g, vorzugsweise im Bereich von 1 bis 10 m²/g, insbesondere 1 bis 5 m²/g.

Als Träger kommen solche fasrigen Materialien in Frage, die sich für die Modifizierung ihrer Oberfläche mit Ionenaustauscherfunktionen eignen. Ein geeignetes Material für den Träger sind beispielsweise Mikrofasern, wobei Mikroglasfasern bevorzugt sind. Solche Materialien weisen eine porenlose Oberfläche auf.

In einer bevorzugten Ausführungsform kann das fasrige Material vor seiner Verwendung als Träger einer Säure- oder Laugenbehandlung unterzogen werden.

Der fasrige Träger kann als Masse, die direkt für die Chromatographie eingesetzt werden kann, vorliegen. Es ist allerdings auch möglich, die Masse für die Trennung in geeignete Formen weiter zu verarbeiten.

Es hat sich herausgestellt, dass für viele Anwendungen das erfindungsgemäße Chromatographiematerial vorteilhafterweise einen Träger umfasst, der in Form einer Membran ausgebildet ist. Für eine geeignete Kapazität des Chromatographiematerials ist es bevorzugt, dass die Membran unabhängig von der Gesamtoberfläche eine Dicke von mindestens 0,05 mm aufweist.

In einer bevorzugten Ausführungsform des erfindungsgemäßen Chromatographiematerials liegt die Membran einlagig vor. Es ist allerdings auch möglich, die Membran mehrlagig vorzusehen, je nach gewünschter Kapazität der Trennung.

Der Träger des erfindungsgemäßen Chromatographiematerials ist mit einem Silanisierungsreagenz umgesetzt. Als Silanisierungsreagenz kann beispielsweise ein solches verwendet werden, das in der WO 91/05606 beschrieben ist. Ebenfalls können nach bekannten Verfahren an der stationären Phase Anionenaustauschergruppen oder Kationenaustauschergruppen angebracht werden. Ein Beispiel dafür ist in der W091/05606 beschrieben.

Das erfindungsgemäße Chromatographiematerial weist den Vorteil auf, dass mit ihm in kürzester Zeit ein Trennvorgang von Nukleinsäuregemischen durchgeführt werden kann. Der Grund ist darin zu sehen, dass auch bei starken Kräften, z. B. Vakuum, auf das erfindungsgemäße Chromatographiematerial, das eine höhere Materialdichte als herkömmliche Chromatographiematerialien aufweist, eine hinreichende Retentionszeit zur Trennung der Nukleinsäuren gegeben ist. Aufgrund der erzeugbar hohen Materialdichte kann die Trennung der Nukleinsäuren mit einem sehr geringen Bettvolumen durchgeführt werden. Wasch- und Elutionsvolumina sind dementsprechend gering.

Mit dem erfindungsgemäßen Chromatographiematerial wird die Trennung von Nukleinsäuregemischen mit höchster Akkuratheit und auch Reinheit der zu gewinnenden Fraktionen durchgeführt. Dieses zeigt sich insbesondere im Vergleich mit den herkömmlichen körnigen Chromatographiematerialien. Zu diesem Zweck wurden Nukleinsäuregemische vor der Trennung in RNA und DNA und nach der Trennung über dem erfindungsgemäßen Chromatographiematerial und zwei herkömmlichen Chromatographiematerialien auf eine Agarosegel laufen gelassen.

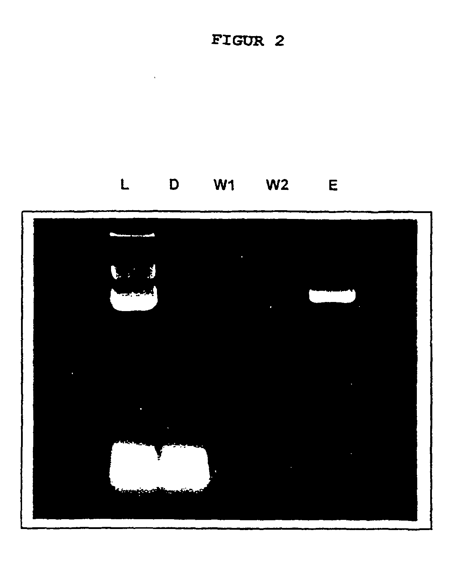

Fig. 2 zeigt die Auftrennung der Nukleinsäuregemischkomponenten mit dem erfindungsgemäßen Chromatographiematerial. Die einzelnen Spuren zeigen die Durchläufe über die Säule:
Spur L: cleared lysate vor der Trennung
Spur D: Säulendurchlauf
Spur W 1: 1. Waschung
Spur W 2: 2. Waschung
Spur E: Elution

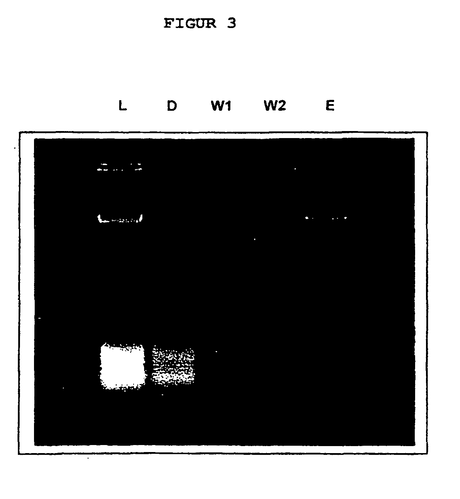

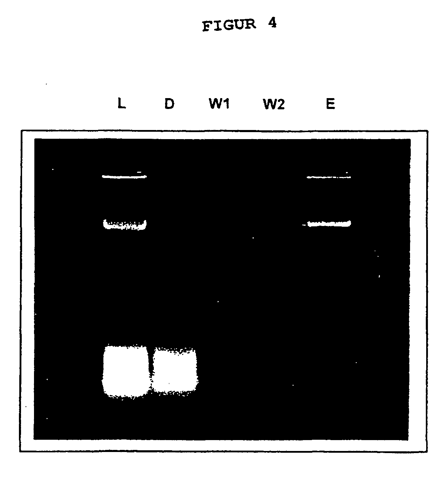

Das gleiche Experiment wurde mit herkömmlichen Chromatographiematerialien durchgeführt. Dazu wird auf die Fig. 3 und 4 verwiesen. In den Spuren wurden die gleichen Säulendurchläufe aufgetragen.

Aus Fig. 2 ist zu erkennen, dass im Eluat in Spur E die RNA von der Plasmid-DNA vollständig getrennt ist. Es ist des weiteren deutlich zu erkennen, dass die einzelnen Durchläufe vor der Elution keinen DNA-Verlust mit sich bringen.

Die Fig. 3 und 4 zeigen die Auftrennung von Nukleinsäuregemischkomponenten mit herkömmlichen, körnigen Chromatographiematerialien. Insbesondere bei der Reinigung in Fig. 3 ist ein starker DNA-Verlust in Spur E zu verzeichnen. Des Weiteren kommt es zu einer schlechten Abreicherung von RNA, was in Spur W 2 zu erkennen ist, wo noch deutliche Mengen an RNA im zweiten Waschdurchgang mitlaufen.

Eine schlechte Abreicherung ist auch bei Verwendung eines weiteren herkömmlichen Chromatographiematerials in Fig. 4 zu erkennen. Die Spur W 2 führt noch erhebliche RNA in der zweiten Waschung.

Ein weiterer Vorteil gegenüber der herkömmlichen körnigen Chromatographiematerialien besteht darin, dass im Eluat E keinerlei Partikel des Chromatographiematerials (fines) enthalten sind. Das führt zu einer erheblichen Verbesserung der Qualität der gewonnenen Nukleinsäuren.

Das erfindungsgemäße Verfahren zur Trennung von Nukleinsäuregemischen unter Verwendung des erfindungsgemäßen Chromatographiematerials zeichnet sich dadurch aus, dass dieses unter Einwirken einer Kraft durchgeführt wird.

In einer bevorzugten Ausführungsform wird das Verfahren unter Anlegung eines Vakuums durchgeführt.

Beispielsweise wird nach Auftragung der Nukleinsäuregemischprobe auf das erfindungsgemäße Chromatographiematerial ein Vakuum angelegt, das eine Trennung von etwa innerhalb 20 Sekunden herbeiführt. Dieses steht im krassen Gegensatz zu den herkömmlichen Kieselgelsäulen, die mit mindestens dem 10-fachen Bettvolumen im Gravity-flow eine Trenndauer von mindestens 20 Minuten erfordern.

Das erfindungsgemäße Chromatographiematerial kann in praktisch allen chromatographischen Verfahren eingesetzt werden. Dazu zählen die Säulenchromatographie, Trennungen in Spincolums und Spincups oder Trennungen im Batch-Verfahren, bei denen das Chromatographiematerial in Suspension vorliegt oder an Reaktionsgefäßen, Mikrotiterplatten, Pipettenspitzen, Rührstäbchen oder Teststreifen fixiert ist.

Bei der chromatographischen Trennung in Spincups wird sich die Zentrifugalkraft zu Nutze gemacht, indem man die in Spincups gefüllte Probe in der Zentrifuge chromatographisch auftrennt.

Mit dem erfindungsgemäßen Chromatographiematerial kann jedes Nukleinsäuregemisch äußerst effektiv aufgetrennt werden. So ist es möglich, aus Gemischen, die neben großen Mengen an RNA nur sehr geringe Mengen an DNA enthalten, die DNA mit höchster Reinheit zu isolieren. Es kann dabei völlig auf die Verwendung toxischer Substanzen, wie Phenol, Chloroform oder Ethidiumbromid verzichtet werden. Des Weiteren können die Trennungen gänzlich unter Vermeidung von RNAse durchgeführt werden.

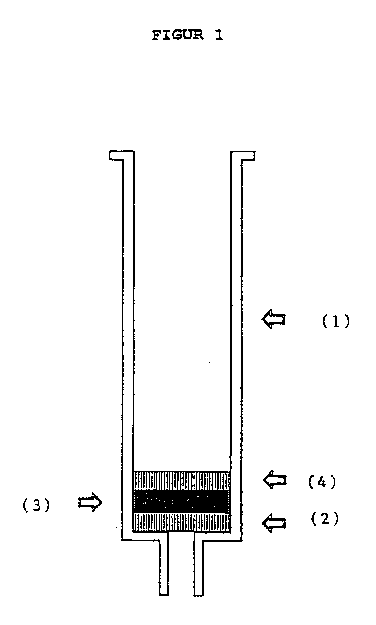

In Fig. 1 ist ein Beispiel für eine Säule, die mit dem erfindungsgemäßen Chromatographiematerial ausgestattet ist, gezeigt. In einer handelsüblichen Kunststoffsäule (1) mit einem nach unten verjüngenden Ausgang zum Anlegen eines Vakuums wird eine Unterfritte (2) mit einer Dicke von 1 mm vorgesehen, die mit dem Ausgang abschließt. Darauf wird das erfindungsgemäße Chromatographiematerial (3) in Form einer Mikroglasfasermembran mit einer Dicke von 2 mm aufgebracht. Zum Abschluss wird darauf eine Oberfritte (4) mit einer Dicke von 1 mm vorgesehen.

Ahnliche Anordnungen finden sich bei allen anderen Chromatographieverfahren, wie z. B. in Spin-cups und auf Mikrotiterplatten (96-Well-Plates).

Das erfindungsgemäße Verfahren zur Trennung von Nukleinsäuregemischen unter Verwendung des erfindungsgemäßen Chromatographiematerials erfolgt in einem einfachen Stufengradienten durch Waschen der mit dem Nukleinsäuregemisch beladenen Anordnung und anschließendes Eluieren der gewünschten Nukleinsäure mit einer geeigneten gepufferten Salzlösung. Der überwiegende Teil der RNA wird während der Bindung der DNA an das erfindungsgemäße Chromatographiematerial abgetrennt, wobei noch verbleibende RNA während des Waschvorgangs herausgespült wird. Somit ist eine Behandlung mit RNAse nicht notwendig.

Wenn die chromatographische Trennung beispielsweise in einer Säule oder Mikrotiterplatte durchgeführt wird, dann wird unter Anlegung eines Vakuums in kürzester Zeit, d. h., in etwa 20 Sekunden, eine Trennung auf der Anordnung der Komponenten durchgeführt. Ebenso erreicht man eine Trennung mit ausgezeichneter Effizienz in Spincups, die man in eine Zentrifuge einsetzt, wobei dann durch die Zentrifugalkraft in einem kurzen Zeitraum, wie etwa 20 Sekunden, die Trennung durchgeführt wird.

Das erfindungsgemäße Chromatographiematerial kann auf verschiedene Weise in den Handel gebracht werden. Beispielsweise ist es möglich, das erfindungsgemäße Chromatographiematerial in der gewünschten Anordnung als Kit zusammen mit dem für die Chromatographie benötigten Equipment, z.B. mit den Puffern, anzubieten. Andere Handelsformen sind selbstverständlich mit eingeschlossen.

Die Erfindung wird anhand der nachfolgenden Beispiele, allerdings ohne Einschränkung darauf, veranschaulicht.

### Beispiele

### Beispiel 1: Anzucht der Bakterienkulturen

Für die Plasmidpräparationen werden E.coli-Kulturen entsprechend gängiger mikrobiologischer Praxis angezogen. Am Tag 1 erfolgt ein Vereinzelungsausstrich aus einer tiefgefrorenen Stock-Kultur auf einem selektiven Medium (z. B. LB-Agar mit Ampicillin als Antibiotikum). Nach einer Übernachtinkubation bei 37°C wird am Tag 2 eine gut gewachsene Einzelkolonie auf 50 - 300 ml Flüssigmedium (z. B. LB), dem das entsprechende Antibiotikum zugesetzt worden ist, inokuliert. Nach einer weiteren Übernachtinkubation bei 37°C auf dem Schüttler bei guter Belüftung (200 - 300 rpm) wird ggf. auf noch größere Kulturvolumina aufgestockt oder die gewachsene Kultur wird direkt abgeerntet. Im Falle des Aufstockens wird die entsprechende mit Antibiotikum versetzte Menge an frischem Flüssigmedium mit 1 % ihres Volumens mit der Kultur des Vortages inokuliert und für eine weitere Übernachtinkubation bei 37°C auf dem Schüttler bei 200 - 300 rpm inkubiert.

Für eine Minipräparation werden 1 - 3 ml Menge Kultur mit High-Copy-Plasmid oder 5 - 20 ml Menge Kultur mit Low-Copy-Plasmid verwendet. Bei Midi- bzw. Maxipräparationen werden entsprechend größere Mengen verwendet.

### Beispiel 2: Isolierung der DNA aus den Bakterien

Die in Beispiel 1 angegebene Menge Bakterienkultur für eine Minipräparation wird in einem geeigneten Zentrifugationsgefäß für 3 Minuten bei 13.000 x g abzentrifugiert und das überstehende Medium wird komplett verworfen. Eventuell vom Rand des Zentrifugationsgefässes zurücklaufendes Medium wird mit der Pipette entfernt und ebenfalls verworfen.

Die pelletierten Bakterien werden durch Vortexen in 0,4 ml Puffer aus 50 mM Tris-HCl (pH 8,0)/10 mM EDTA/100 µg/ml RNAse vollständig resuspendiert. Es dürfen keine Zellklumpen oder - aggregate mehr zu erkennen sein.

Die suspendierten Zellen werden durch Zugabe von 0,4 ml Puffer aus 200 mM NaOH/ 0,1 % (w/v) SDS lysiert. Die suspendierten Zellen werden mit dem Lysepuffer durch mehrmaliges Invertieren gemischt bis eine homogene Phase entstanden ist. Diese Phase ist durch die ausgetretene genomische Bakterien-DNA sehr viskos. Es wird für maximal 5 Minuten bei Raumtemperatur inkubiert.

Das Lysegemisch wird durch Zugabe von 0,4 ml Puffer aus 3.1-3.4 M Kaliumacetat (pH 5,5 mit Essigsäure) neutralisiert. Nach Zugabe des Puffers wird wiederum durch mehrmaliges Invertieren gemischt bis eine homogene Phase entstanden ist. Diese Phase ist jetzt wieder dünnflüssig. Es dürfen keine viskosen Reste des Zelllysates mehr vorhanden sein.

Das bei der Neutralisation ausgefallene Präzipität aus bakteriellen Proteinen und Zelltrümmern wird durch eine 10-minütige Zentrifugation mit ≥ 13.000 x g bei Raumtemperatur abzentrifugiert und der klare Überstand ("Cleared Lysate") abpipettiert.

### Beispiel 3: Herstellung einer Säule mit dem erfindungsgemäßen Chromatographiematerial

5g porenlose Glasfaser mit einer spezifischen Oberfläche von 5 m²/g werden in 750 ml trockenem Xylol mit 60 g 3-Glycidoxypropyltrimethoxysilan und 0,13 ml Triethylamin versetzt. Die Reaktionsmischung wird durch dreimaliges Anlegen eines Vakuums und anschließendem Belüften mit Stickstoff entgast und bei 130°C unter Ausschluss von Luft und Feuchtigkeit für 4 Stunden erhitzt. Es wird abfiltriert und mit Xylol und Tetrahydrofuran gewaschen. Die modifizierte Glasfaser wird bei 50 °C im Vakuum getrocknet.

Das Produkt wird anschließend mit 750 ml und 42 g Diethylamin versetzt und 18 Stunden unter Rückfluss erhitzt. Das Produkt wird mit Dioxan und Methanol gewaschen und bei 70 °C im Vakuum getrocknet. Die so modifizierte Glasfasermasse mit Anionenaustauscherfunktion wird nun zu einer Anionenaustauschermembran weiterverarbeitet. Dabei wird die Glasfasermasse in Aceton aufgeschlämmt und in die entsprechende Form gewalzt oder gegossen und abschließend getrocknet. Eine Membran entsprechend dem Säulendurchmesser wird dann geschnitten und in die Anordnung nach Fig. 1 eingesetzt.

### Beispiel 4: Trennung der Bakterien-DNA

Die Säule nach Beispiel 3 wird auf eine passende Vakuumkammer (z. B. VacMan, Promega) aufgesetzt. Die IonenaustauscherMembran wird mit 2 ml Puffer aus 100 mM NaAc/HAc (pH 5,0)/600 mM NaCl äquilibriert. Dazu wird der Puffer in die Säule pipettiert und durch das Anlegen eines Wasserstrahl-Vakuums durch die Membran vollständig durchgezogen. Die Vakuumpumpe bleibt so lange eingeschaltet, bis erkennbar keine Flüssigkeit mehr von der Membran abgesaugt wird. Danach wird das Vakuum abgeschaltet.

Die Säule wird mit dem "Cleared Lysate" aus Beispiel 2 beladen und durch das Anlegen des Wasserstrahl-Vakuums durch die Membran vollständig durchzogen. Die Vakuumpumpe bleibt wieder so lange eingeschaltet, bis erkennbar keine Flüssigkeit mehr von der Membran abgesaugt wird. Danach wird das Vakuum abgeschaltet.

Für die Entfernung unspezifisch gebundener Komponenten wird die Säule mit 2,5 ml Puffer aus 100 mM NaAc/HAc (pH 5,0)/600 mM NaCl gewaschen. Dazu wird der Puffer in die Säule pipettiert und durch das Anlegen eines Wasserstrahl-Vakuums durch die Membran vollständig durchzogen. Die Vakuumpumpe bleibt so lange eingeschaltet, bis erkennbar keine Flüssigkeit mehr von der Matrix abgesaugt wird. Danach wird das Vakuum abgeschaltet. Dieser Waschschritt wird im Falle der Mini- und der Midi-Präparation einmal wiederholt.

Die Säule wird von der Vakuumkammer abgelöst und die an die Membran gebundene Plasmid-DNA durch Zugabe von 0,8 ml Puffer 100 mM Tris-HCl (pH 8,5)/1250 mM NaCl direkt in ein geeignetes Gefäß eluiert. Dazu wird der Puffer in die Säule pipettiert und mit Hilfe eines passenden Stempels durch die Membran manuell durchgedrückt. Hierbei sollte der Elutionspuffer in einer raschen Tropfenfolge, aber keinesfalls als Strahl durchgedrückt werden. Die einzelnen Tropfen müssen mit dem bloßen Auge noch zu erkennen sein.

Die Eluate werden mit 0,7 Vol. Isopropanol (Raumtemperatur) versetzt und gut gemischt. Die so präzipitierte Plasmid-DNA wird für mindestens 30 Minuten bei ≥ 13.000 x g und 4°C abzentrifugiert und der Überstand verworfen. Die pelletierte DNA wird einmal mit 80 %igem Ethanol gewaschen, wieder abzentrifugiert, anschließend getrocknet (entweder durch Stehenlassen bei Raumtemperatur oder im Vakuum) und die getrocknete DNA wird schließlich in einer geeigneten Menge TE-Puffer oder Wasser für 10 Minuten bei 37°C gelöst.

Die gelöste Plasmid-DNA wird spektrophotometrisch vermessen und auf einem Agarosegel analysiert.

Fig. 2 zeigt die Auftrennung der Gemischkomponenten auf einem 1 %igen Agarosegel mit einem TAE-Puffer, pH 8,3. In den einzelnen Spuren wurden folgende Komponenten aufgetragen:
Spur L: Cleared Lysate vor der Trennung
Spur D: Säulendurchlauf
Spur W1: Erste Waschung
Spur W2: Zweite Waschung
Spur E: Elution

In den Spuren L, D und W1 ist deutlich zu erkennen, dass in der Präparation immer noch RNA enthalten ist. Eine vollständige Auftrennung der Nukleinsäure-Gemischkomponenten ist aber deutlich am Eluat in Spur E zu erkennen, die lediglich die Plasmid-DNA ohne Verunreinigung mit RNA zeigt.

### Beispiel 5: Trennung von Bakterien-DNA

Es wurde die gleiche Bakterien-DNA wie in Beispiel 2 über eine Säule der Firma Macherey-Nagel (NUCLEOBOND Kits), die ein herkömmliches Chromatographiematerial enthielt, gegeben und nach Herstellerangaben aufgetrennt. Die Ergebnisse sind in Fig. 3 gezeigt.

### Beispiel 6: Trennung von Bakterien-DNA

Es wurde ein herkömmliches Chromatographiematerial der Firma Qiagen (Qiagen Plasmid Kits) verwendet, um die Bakterien von Beispiel 2 aufzutrennen. Es wurde dabei nach Herstellerangaben gearbeitet. Die Ergebnisse sind in Fig. 4 aufgezeigt.

## Patentansprüche

1. Chromatographiematerial zur Trennung von Nukleinsäuregemischen mit einem Träger und darauf aufgebrachten Ionenaustauscherfunktionen, **dadurch gekennzeichnet, dass** der Träger aus Mikroglasfasern zusammengesetzt ist.

2. Chromatographiematerial nach Anspruch 1, **dadurch gekennzeichnet, dass** die spezifische Oberfläche des Trägers 0,05 bis 50 m²/g beträgt.

3. Chromatographiematerial nach mindestens einem der Ansprüche 1 und/oder 2, **dadurch gekennzeichnet, dass** das fasrige Material in Form einer Masse vorliegt.

4. Chromatographiematerial nach Anspruch 3, **dadurch gekennzeichnet, dass** die Masse als Membran ausgebildet ist.

5. Chromatographiematerial nach Anspruch 4, **dadurch gekennzeichnet, dass** die Membran eine Dicke von mindestens 0,05 mm aufweist.

6. Chromatographiematerial nach Anspruch 5, **dadurch gekennzeichnet, dass** die Membran einlagig vorliegt.

7. Chromatographiematerial nach Anspruch 6, **dadurch gekennzeichnet, dass** die Membran mehrlagig vorliegt.

8. Chromatographiematerial nach mindestens einem der Ansprüche 1 bis 7, **dadurch gekennzeichnet, dass** der Träger mit einem Silanisierungsreagenz umgesetzt ist.

9. Chromatographiematerial nach Anspruch 8, **dadurch gekennzeichnet, dass** Anionenaustauschergruppen über das Silanisierungsreagenz gekoppelt sind.

10. Chromatographiematerial nach Anspruch 8, **dadurch gekennzeichnet, dass** Kationenaustauschergruppen über das Silanisierungsreagenz gekoppelt sind.

11. Verfahren zur Trennung von Nukleinsäuregemischen unter Verwendung eines Chromatographiematerials nach mindestens einem der Ansprüche 1 bis 10, **dadurch gekennzeichnet, dass** die chromatographische Trennung mittels Einwirken einer Kraft durchgeführt wird.

12. Verfahren nach Anspruch 11, **dadurch gekennzeichnet, dass** ein Vakuum angelegt wird.

13. Verfahren nach den Ansprüchen 11 und 12, **dadurch gekennzeichnet, dass** die chromatographische Trennung in einer Säule oder auf Mikrotiterplatten durchgeführt wird.

14. Verfahren nach Anspruch 13, **dadurch gekennzeichnet, dass** die Trennung durch die Zentrifugalkraft erfolgt.

15. Verfahren nach Anspruch 14, **dadurch gekennzeichnet, dass** die Trennung in Spincups in der Zentrifuge erfolgt.

16. Verfahren nach mindestens einem der Ansprüche 11 bis 15, **dadurch gekennzeichnet, dass** die chromatographische Trennung in einem Stufengradienten durchgeführt wird.

17. Verfahren nach mindestens einem der Ansprüche 11 bis 16, **dadurch gekennzeichnet, dass** keine RNAse verwendet wird,

18. Kit zur Trennung von Nukleinsäuregemischen, das ein Chromatographiematerial nach mindestens einem der Ansprüche 1 bis 10 enthält.

## Claims

1. Chromatography material for the separation of nucleic acid mixtures with a support and ion exchanger functions applied thereto, **characterised in that** the support is composed of microglass fibres.

2. Chromatography material according to Claim 1, **characterised in that** the specific surface of the support is 0.05 to 50 m²/g.

3. Chromatography material according to at least one of Claims 1 and/or 2, **characterised in that** the fibrous material is in the form of a mass.

4. Chromatography material according to Claim 3, **characterised in that** the mass is constructed as a membrane.

5. Chromatography material according to Claim 4, **characterised in that** the membrane has a thickness of at least 0.05 mm.

6. Chromatography material according to Claim 5, **characterised in that** the membrane is in the form of a single layer.

7. Chromatography material according to Claim 6, **characterised in that** the membrane is in the form of multiple layers.

8. Chromatography material according to at least one of Claims 1 to 7, **characterised in that** the support has been reacted with a silanising reagent.

9. Chromatography material according to Claim 8, **characterised in that** anion exchanger groups have been coupled via the silanising reagent.

10. Chromatography material according to Claim 8, **characterised in that** cation exchanger groups have been coupled via the silanising reagent.

11. Method for the separation of nucleic acid mixtures using a chromatography material according to at least one of Claims 1 to 10, **characterised in that** the chromatographic separation is carried out by means of the action of a force.

12. Method according to Claim 11, **characterised in that** a vacuum is applied.

13. Method according to Claims 11 and 12, **characterised in that** the chromatographic separation is carried out in a column or on microtitre plates.

14. Method according to Claim 13, **characterised in that** the separation takes place by centrifugal force.

15. Method according to Claim 14, **characterised in that** the separation takes place in spin cups in the centrifuge.

16. Method according to at least one of Claims 11 to 15, **characterised in that** the chromatographic separation is carried out in a step gradient.

17. Method according to at least one of Claims 11 to 16, **characterised in that** no RNAse is used.

18. Kit for the separation of nucleic acid mixtures that contains a chromatography material according to at least one of Claims 1 to 10.

## Revendications

1. Matériau de chromatographie pour la séparation de mélanges d'acides nucléiques avec un porteur et des fonctions échangeuses d'ions appliquées dessus, **caractérisé en ce que** le porteur est composé de microfibres de verre.

2. Matériau de chromatographie selon la revendication 1, **caractérisé en ce que** la surface spécifique du porteur est de 0,05 à 50 m²g.

3. Matériau de chromatographie selon au moins une des revendications 1 et/ou 2, **caractérisé en ce que** le matériel fibreux est présent sous la forme d'une masse.

4. Matériau de chromatographie selon la revendication 3, **caractérisé en ce que** la masse est formée en membrane.

5. Matériau de chromatographie selon la revendication 4, **caractérisé en ce que** la membrane présente une épaisseur d'au moins 0,05 mm.

6. Matériau de chromatographie selon la revendication 5, **caractérisé en ce que** la membrane est monocouche.

7. Matériau de chromatographie selon la revendication 6, **caractérisé en ce que** la membrane est multicouche.

8. Matériau de chromatographie selon au moins l'une des revendications 1 à 7, **caractérisé en ce que** le porteur est transformé avec un réactif aux silanes.

9. Matériau de chromatographie selon la revendication 8, **caractérisé en ce que** des groupes échangeurs d'anions sont couplés par le réactif aux silanes.

10. Matériau de chromatographie selon la revendication 9, **caractérisé en ce que** des groupes échangeurs de cations sont couplés par le réactif aux silanes.

11. Procédé de séparation de mélanges d'acides nucléiques utilisant un matériau de chromatographie selon au moins l'une des revendications 1 à 10, **caractérisé en ce que** la séparation chromatographique est réalisée par l'action d'une force.

12. Procédé selon la revendication 11, **caractérisé en ce qu'**un vide est appliqué.

13. Procédé selon les revendications 11 et 12, **caractérisé en ce que** la séparation chromatographique est réalisée dans une colonne ou sur des plaques de microtitration.

14. Procédé selon la revendication 13, **caractérisé en ce que** la séparation a lieu par force centrifuge.

15. Procédé selon la revendication 14, **caractérisé en ce que** la séparation a lieu dans des coupelles spin dans la centrifugeuse.

16. Procédé selon au moins l'une des revendications 11 à 15, **caractérisé en ce que** la séparation chromatographique est réalisée dans un gradient à paliers.

17. Procédé selon au moins l'une des revendications 11 à 16, **caractérisé en ce qu'**aucune RNase n'est utilisée.

18. Kit de séparation de mélanges d'acides nucléiques qui contient un matériau de chromatographie selon au moins l'une des revendications 1 à 10.
